# EUROPEAN PATENT APPLICATION

(11) **EP 4 450 079 A1**
(43) Date of publication of application: **23.10.2024**
(21) Application number: 22907769.8
(22) Date of filing: 29.11.2022
(51) Int. Cl.: A61K 38/08, A61P 27/02, C07K 7/06

(54) **COMPOSITION FOR TREATING MACULAR DEGENERATION COMPRISING NOVEL PEPTIDE**

(30) Priority: 13.12.2021 KR 20210178126; 21.09.2022 KR 20220119589
(71) Applicant: EyebioKorea, Inc., Busan 47397 (KR)
(72) Inventor: CHO, Yunseok, Suwon-si Gyeonggi-do 16323 (KR); AHN, Byul Nim, Busan 47327 (KR)
(74) Representative: Callaghan, Dayle Anne
(86) International application number: PCT/KR2022/019093
(87) International publication number: WO 2023/113300

(57) **Abstract**

The present invention relates to a composition for preventing or treating macular degeneration, comprising a novel peptide. Specifically, since the peptide of the present invention has an excellent effect of inhibiting choroidal neovascularization, which is a major cause of macular degeneration, and shows excellent stability and solubility, the peptide can be developed as an excellent therapeutic agent for macular degeneration.

## Description

### Technical Field

The present invention relates to a composition for preventing or treating macular degeneration, comprising a novel peptide. Specifically, the peptide of the present invention has an effect of inhibiting choroidal neovascularization, which is a major cause of macular degeneration, and has excellent stability and solubility.

### Background Art

The macula is the part that plays the most important role in the neural layer within the eye called the retina. It is the central part of the retina with a radius of about 1.5 mm, and photoreceptors that can sense light are concentrated in this area. Therefore, the macula is the center of the retina and is responsible for most of vision. Macular degeneration is a disease in which the macula degenerates due to aging, genetic factors, toxicity, inflammation, and the like. As the macula degenerates, vision decreases, and in severe cases, vision may be lost completely.

Macular degeneration is largely divided into non-exudative (dry) and exudative (wet). Non-exudative macular degeneration accounts for 80-90% of all macular degeneration and does not significantly affect vision in most cases, except in the late stage when retinal atrophy and choroidal atrophy occur. However, caution is needed as it can progress to exudative macular degeneration. Exudative macular degeneration accounts for approximately 10-20% of all macular degeneration, but if left untreated, vision rapidly deteriorates, leading many patients to blindness within two years of diagnosis.

Exudative macular degeneration is a stage in which choroidal neovascularity occurs, and has a very poor vision prognosis, making it the disease with the highest incidence of blindness in people over 65 years of age. The vascular layer called the choroid serves to supply nutrients to the retinal layer and remove metabolites from retinal cells. However, due to causes such as aging, there are cases where the blood vessels of the choroid penetrate into the retinal cells and form abnormally. In this case, the abnormal blood vessels are referred to as choroidal neovascularity. Because these blood vessels are abnormal, they are very weak and prone to bursting, causing exudate and blood to flow out and damage the macular area.

Treatment of macular degeneration involves, for non-exudative macular degeneration, taking antioxidant vitamins known to slow the progression of macular degeneration and treating hypertension, hyperlipidemia and the like, which are risk factors for macular degeneration. For exudative macular degeneration, treatment includes thermal laser photocoagulation, photodynamic therapy, antibody injection, vitrectomy, and the like, but there is still no complete treatment and active research is in progress. Recently, the method of injecting anti-vascular endothelial growth factor antibodies (anti-VEGF antibodies) such as aflibercept, ranibizumab, and bevacizumab into the eye has been mainly used, but has disadvantages such as repeated administration and high cost.

Accordingly, the present inventors conducted research on substances that can effectively treat macular degeneration. As a result, the present inventors identified a novel peptide that not only has excellent stability and solubility, but also has an excellent effect of inhibiting choroidal neovascularization, thereby completing the present invention.

### Prior Art Document

### Patent Document

(Patent Document 1) Korean Patent Application No. 10-2020-0060397

### Detailed Description of Invention

### Technical Problem

An object of the present invention is to provide a pharmaceutical composition for preventing or treating macular degeneration, comprising a peptide represented by formula 1.

In addition, another object of the present invention is to provide a pharmaceutical composition for preventing or treating macular degeneration, comprising a peptide consisting of the amino acid sequences of SEQ ID NOs: 1 to 45.

### Solution to Problem

The present invention provides a pharmaceutical composition for preventing or treating macular degeneration, comprising a peptide represented by the following formula 1:

[Formula 1] X₁-X₂-X₃-X₄-X₅-X₆-X₇

in the formula, X₁ is an amino acid selected from the group consisting of proline, trans-4-hydroxy-L-proline, trans-4-hydroxy-D-proline, cis-4-fluoro-L-proline, 4,4,-difluoro-L-proline, 4-methylene-L-proline, 4,4-dimethyl-L-proline, and trans-4-amino-L-proline,
X₂ is glycine,
X₃ is glutamine or D-glutamine,
X₄ is an amino acid selected from the group consisting of aspartate, D-aspartate, glutamate, and D-glutamate,
X₅ is a non-polar amino acid,
X₆ is absent, or leucine or D-leucine, and
X₇ is absent, or an amino acid selected from the group consisting of alanine, D-alanine, and alanine-isopropyl ester,
wherein when X₆ is absent, X₇ is also absent.

In one embodiment, in the formula, X₁ may be an amino acid selected from the group consisting of proline, trans-4-hydroxy-L-proline, trans-4-hydroxy-D-proline, cis-4-fluoro-L-proline, 4,4,-difluoro-L-proline, 4-methylene-L-proline, 4,4-dimethyl-L-proline, and trans-4-amino-L-proline,
X₂ may be glycine,
X₃ may be glutamine or D-glutamine,
X₄ may be an amino acid selected from the group consisting of aspartate, D-aspartate, glutamate, and D-glutamate,
X₅ may be an amino acid selected from the group consisting of glycine, phenyl-glycine, alanine, valine, leucine, tert-leucine, and 2-aminoisobutyric acid,
X₆ may be absent, or leucine or D-leucine, and
X₇ may be absent, or an amino acid selected from the group consisting of alanine, D-alanine, and alanine-isopropyl ester, wherein when X₆ is absent, X₇ is also absent.

In one embodiment, in the formula, X₁ may be an amino acid selected from the group consisting of proline, trans-4-hydroxy-L-proline, trans-4-hydroxy-D-proline, cis-4-fluoro-L-proline, 4,4,-difluoro-L-proline, 4-methylene-L-proline, 4,4-dimethyl-L-proline, and trans-4-amino-L-proline,
X₂ may be glycine,
X₃ may be glutamine or D-glutamine,
X₄ may be an amino acid selected from the group consisting of aspartate, D-aspartate, glutamate, and D-glutamate,
X₅ may be a non-polar amino acid,
X₆ may be leucine or D-leucine, and
X₇ may be an amino acid selected from the group consisting of alanine, D-alanine, and alanine-isopropyl ester.

In one embodiment, in the formula, X₁ may be an amino acid selected from the group consisting of proline, trans-4-hydroxy-L-proline, trans-4-hydroxy-D-proline, cis-4-fluoro-L-proline, 4,4,-difluoro-L-proline, 4-methylene-L-proline, 4,4-dimethyl-L-proline, and trans-4-amino-L-proline,
X₂ may be glycine,
X₃ may be glutamine or D-glutamine,
X₄ may be an amino acid selected from the group consisting of aspartate, D-aspartate, glutamate, and D-glutamate,
X₅ may be an amino acid selected from the group consisting of glycine, phenyl-glycine, alanine, valine, leucine, tert-leucine, and 2-aminoisobutyric acid,
X₆ may be leucine or D-leucine, and
X₇ may be an amino acid selected from the group consisting of alanine, D-alanine, and alanine-isopropyl ester.

In one embodiment, in the formula, X₁ may be an amino acid selected from the group consisting of proline, trans-4-hydroxy-L-proline, trans-4-hydroxy-D-proline, cis-4-fluoro-L-proline, 4,4,-difluoro-L-proline, 4-methylene-L-proline, 4,4-dimethyl-L-proline, and trans-4-amino-L-proline,
X₂ may be glycine,
X₃ may be glutamine or D-glutamine,
X₄ may be glutamate or D-glutamate,
X₅ may be 2-aminoisobutyric acid,
X₆ may be leucine or D-leucine, and
X₇ may be an amino acid selected from the group consisting of alanine, D-alanine, and alanine-isopropyl ester.

The composition of the present invention may comprise any one peptide selected from the group consisting of the amino acid sequences of SEQ ID NOs: 1 to 45, and preferably, the composition of the present invention may comprise a peptide consisting of the amino acid sequence of SEQ ID NO: 43.

The peptide of the present invention has an effect of inhibiting choroidal neovascularization (CNV)

The macular degeneration of the present invention may be at least one selected from the group consisting of wet macular degeneration, dry macular degeneration, and age-related macular degeneration.

The present invention provides a method for treating macular degeneration, comprising administering a peptide represented by the following formula 1 to a subject with macular degeneration disease:

[Formula 1] X₁-X₂-X₃-X₄-X₅-X₆-X₇

in the formula, X₁ is an amino acid selected from the group consisting of proline, trans-4-hydroxy-L-proline, trans-4-hydroxy-D-proline, cis-4-fluoro-L-proline, 4,4,-difluoro-L-proline, 4-methylene-L-proline, 4,4-dimethyl-L-proline, and trans-4-amino-L-proline,
X₂ is glycine,
X₃ is glutamine or D-glutamine,
X₄ is an amino acid selected from the group consisting of aspartate, D-aspartate, glutamate, and D-glutamate,
X₅ is a non-polar amino acid,
X₆ is absent, or leucine or D-leucine, and
X₇ is absent, or an amino acid selected from the group consisting of alanine, D-alanine, and alanine-isopropyl ester,
wherein when X₆ is absent, X₇ is also absent.

The present invention provides a composition for use in the treatment or prevention of macular degeneration, comprising the peptide represented by the formula 1.

The present invention provides a use of the composition for treating or preventing macular degeneration, comprising the peptide represented by the formula 1.

### Effects of Invention

The peptide of the present invention has excellent stability and solubility and has an excellent effect of inhibiting choroidal neovascularization, which is a major cause of macular degeneration, and thus can be usefully used for the prevention or treatment of macular degeneration.

### Brief Description of Drawings

Figures 1 and 2 show the results obtained by measuring the CNV of the peptide of the present invention by FFA (fundus fluorescein angiography) and the results obtained by calculating the CTF (corrected total fluorescence).
Figures 3 and 4 show the results obtained by measuring the CNV of the peptide of the present invention by OCT (optical coherence tomography) and the results obtained by calculating the size of the CNV lesion.
Figure 5 shows the results obtained by measuring the CNV of the peptide of the present invention by electroretinography.
Figures 6 and 7 show the results obtained by measuring the CNV of the peptide of Example 43 (002-175) of the present invention and an eye drop comprising the same by FFA (fundus fluorescein angiography) and the results obtained by quantifying the vascular leakage.
Figures 8 and 9 show the results obtained by measuring the CNV of the peptide of Example 43 (002-175) of the present invention and an eye drop comprising the same by OCT (optical coherence tomography) and the results obtained by calculating the size of the CNV lesion.
Figures 10 and 11 show the results obtained by measuring the CNV of the eye drop comprising the peptide of Example 43 (002-175) of the present invention by FFA (fundus fluorescein angiography) and the results obtained by calculating the neovascularity site.

### Best Mode for Carrying out the Invention

Hereinafter, with reference to the accompanying drawings, embodiments and examples of the present invention will be described in detail so that those of ordinary skill in the art to which the present invention belongs can easily practice the present invention. However, the present invention may be implemented in various forms and is not limited to the embodiments and examples described herein.

Throughout the present specification, when a certain part "includes" a certain component, it means that other components may be further included, rather than excluding other components, unless otherwise stated.

The present invention provides a pharmaceutical composition for preventing or treating macular degeneration, comprising a peptide represented by the following formula 1:

[Formula 1] X₁-X₂-X₃-X₄-X₅-X₆-X₇

in the formula, X₁ is an amino acid selected from the group consisting of proline, trans-4-hydroxy-L-proline, trans-4-hydroxy-D-proline, cis-4-fluoro-L-proline, 4,4,-difluoro-L-proline, 4-methylene-L-proline, 4,4-dimethyl-L-proline, and trans-4-amino-L-proline,
X₂ is glycine,
X₃ is glutamine or D-glutamine,
X₄ is an amino acid selected from the group consisting of aspartate, D-aspartate, glutamate, and D-glutamate,
X₅ is a non-polar amino acid,
X₆ is absent, or leucine or D-leucine, and
X₇ is absent, or an amino acid selected from the group consisting of alanine, D-alanine, and alanine-isopropyl ester,
wherein when X₆ is absent, X₇ is also absent.

The peptide of the present invention may be a peptide selected from the group consisting of the peptides shown in Table 1 below.

**[Table 1]**

| Example | Peptide No. | Amino acid sequence | | | | | | | SEQ ID NO |
|---|---|---|---|---|---|---|---|---|---|
| 1 | 002-053 | Hyp | Gly | Gln | Asp | Val | | | 1 |
| 2 | 002-074 | Hyp | Gly | Gln | Asp | Leu | | | 2 |
| 3 | 002-075 | Hyp | Gly | Gln | Asp | Ala | | | 3 |
| 4 | 002-103 | Hyp | Gly | D-Gln | Asp | Gly | | | 4 |
| 5 | 002-104 | Hyp | Gly | Gln | D-Asp | Gly | | | 5 |
| 6 | 002-085 | D-Hyp | Gly | Gln | Asp | Gly | | | 6 |
| 7 | 002-076 | Hyp | Gly | Gln | Asp | Val | Leu | | 7 |
| 8 | 002-077 | Hyp | Gly | Gln | Asp | Leu | Leu | | 8 |
| 9 | 002-078 | Hyp | Gly | Gln | Asp | Ala | Leu | | 9 |
| 10 | 002-105 | Hyp | Gly | D-Gln | Asp | Gly | Leu | | 10 |
| 11 | 002-106 | Hyp | Gly | Gln | D-Asp | Gly | Leu | | 11 |
| 12 | 002-107 | Hyp | Gly | Gln | Asp | Gly | D-Leu | | 12 |
| 13 | 002-090 | D-Hyp | Gly | Gln | Asp | Gly | Leu | | 13 |
| 14 | 002-055 | Hyp | Gly | Gln | Asp | Val | Leu | Ala | 14 |
| 15 | 002-080 | Hyp | Gly | Gln | Asp | Leu | Leu | Ala | 15 |
| 16 | 002-081 | Hyp | Gly | Gln | Asp | Ala | Leu | Ala | 16 |
| 17 | 002-084 | Hyp | Gly | Gln | Asp | Gly | Leu | Ala-IPE | 17 |
| 18 | 002-108 | Hyp | Gly | D-Gln | Asp | Gly | Leu | Ala | 18 |
| 19 | 002-109 | Hyp | Gly | Gln | D-Asp | Gly | Leu | Ala | 19 |
| 20 | 002-110 | Hyp | Gly | Gln | Asp | Gly | D-Leu | Ala | 20 |
| 21 | 002-111 | Hyp | Gly | Gln | Asp | Gly | Leu | D-Ala | 21 |
| 22 | 002-086 | ci s-4F-Pro | Gly | Gln | Asp | Gly | | | 22 |
| 23 | 002-087 | trans-4NH₂-Pro | Gly | Gln | Asp | Gly | | | 23 |
| 24 | 002-088 | 4,4-difluoro-Pro | Gly | Gln | Asp | Gly | | | 24 |
| 25 | 002-089 | 4-methylene-Pro | Gly | Gln | Asp | Gly | | | 25 |
| 26 | 002-100 | 4,4-dimethyl Pro | Gly | Gln | Asp | Gly | | | 26 |
| 27 | 002-091 | ci s-4F-Pro | Gly | Gln | Asp | Gly | Leu | | 27 |
| 28 | 002-092 | trans-4NH₂-Pro | Gly | Gln | Asp | Gly | Leu | | 28 |
| 29 | 002-093 | 4,4-difluoro-Pro | Gly | Gln | Asp | Gly | Leu | | 29 |
| 30 | 002-094 | 4-methylene-Pro | Gly | Gln | Asp | Gly | Leu | | 30 |
| 31 | 002-101 | 4,4-dimethyl Pro | Gly | Gln | Asp | Gly | Leu | | 31 |
| 32 | 002-095 | D-Hyp | Gly | Gln | Asp | Gly | Leu | Ala | 32 |
| 33 | 002-096 | ci s-4F-Pro | Gly | Gln | Asp | Gly | Leu | Ala | 33 |
| 34 | 002-099 | 4-methylene-Pro | Gly | Gln | Asp | Gly | Leu | Ala | 34 |
| 35 | 002-102 | 4,4-dimethyl | Gly | Gln | Asp | Gly | Leu | Ala | 35 |
| | | Pro | | | | | | | |
| 36 | 002-130 | Hyp | Gly | Gln | Glu | Gly | | | 36 |
| 37 | 002-170 | Hyp | Gly | Gln | Glu | Val | | | 37 |
| 38 | 002-167 | Hyp | Gly | Gln | Glu | Leu | Leu | | 38 |
| 39 | 002-168 | Hyp | Gly | Gln | Glu | Val | Leu | | 39 |
| 40 | 002-132 | Hyp | Gly | Gln | Glu | Gly | Leu | Ala | 40 |
| 41 | 002-165 | Hyp | Gly | Gln | Glu | Leu | Leu | Ala | 41 |
| 42 | 002-166 | Hyp | Gly | Gln | Glu | Val | Leu | Ala | 42 |
| 43 | 002-175 | Hyp | Gly | Gln | Glu | Aib | Leu | Ala | 43 |
| 44 | 002-176 | Hyp | Gly | Gln | Glu | tert-Leu | Leu | Ala | 44 |
| 45 | 002-177 | Hyp | Gly | Gln | Glu | phenyl-Gly | Leu | Ala | 45 |

As used herein, the term "macular degeneration" refers to a disease in which vision decreases as the macula deteriorates in function due to aging, genetic factors, toxicity, inflammation, and the like, and in severe cases, vision may be completely lost.

In the present invention, the macular degeneration may include wet macular degeneration, dry macular degeneration, and age-related macular degeneration, but is not limited thereto.

As used herein, the term "choroidal neovascularization (CNV)" refers to the process of generating new choroidal capillaries. The choroid is a highly vascular membrane of the eye and refers to a complex network of fine capillaries that supply nutrients to the iris and retina located in the periphery where they are found. Pathologically, choroidal neovascularization refers to the violation of the subretinal space due to abnormal blood vessels with stems formed from the choroid and structural deformation and destruction of Bruch's membrane.

As used herein, the term "neovascularization" refers to the process of forming new blood vessels, that is, the development of new blood vessels into cells, tissues, or organs, and "neovascularity" refers to blood vessels newly created through the neovascularization process. As used herein, "neovascularization" and "neovascularity" can be used interchangeably.

The CNV animal model used in the present invention is a mouse model in which lesions are induced by irradiating a laser to the choroid of C57BL/6 mice using a laser slit system. The laser-induced CNV model, first described in 1979, uses photocoagulation to disturb Bruch's membrane to induce the growth of choroidal neovascularization into the retina area, and it is similar in development to neovascular disease in the human retina, that is, wet macular degeneration (wet AMD), in that neovascularity arises from the choroid. In addition, the laser-induced CNV model was successful in predicting the clinical efficacy of anti-vascular endothelial growth factor (VEGF) treatment for neovascular AMD, and is an animal model used in the development stage of existing therapeutic agents (Gong Y, Li J, Sun Y, Fu Z, Liu C-H, Evans L, et al. (2015) Optimization of an Image-Guided Laser-Induced Choroidal Neovascularization Model in Mice. PLoS ONE 10(7)).

In the present invention, Pro is proline,
Hyp is trans-4-hydroxy-L-proline,
D Hyp is trans-4-hydroxy-D-proline,
cis-4F-Pro is cis-4-fluoro-L-proline,
4,4-difluoro-Pro is 4,4,-difluoro-L-proline,
4-methylene-Pro is 4-methylene-L-proline,
4,4-dimethyl Pro is 4,4-dimethyl-L-proline,
trans-4NH₂-Pro is trans-4-amino-L-proline,
Gly is glycine,
Gln is glutamine,
D-Gln is D-glutamine,
Ala is alanine,
D-Ala is D-alanine,
Val is valine,
Leu is leucine,
D-Leu is D-leucine,
Ile is isoleucine,
Met is methionine,
tert-Leu is tert-leucine or L-α-tert-butylglycine,
Aib is 2-aminoisobutyric acid,
isopropyl ester (IPE) is a derivative in which the terminal amino acid group is substituted with isopropyl ester.
Ser is serine,
Thr is threonine,
Cys is cysteine,
Asn is asparagine,
Phe is phenylalanine,
Tyr is tyrosine,
Trp is tryptophan,
Lys is lysine,
Arg is arginine,
His is histidine,
Asp is aspartate or aspartic acid,
D-Asp is D-aspartate or D-aspartic acid,
Glu is glutamate or glutamic acid, and
D-Glu is D-glutamate or D-glutamic acid.

In the present invention, a polar amino acid refers to an amino acid whose a side chain has polarity in the chemical structure of the amino acid, and includes Thr, Ser, Cys, Asn, Gln, Asp, Glu, His, Lys, and Arg, but is not limited thereto.

In the present invention, a non-polar amino acid refers to an amino acid whose a side chain does not have polarity in the chemical structure of the amino acid, and includes Met, Leu, Pro, Ala, Gly, Val, Ile, and Aib, but is not limited thereto.

As used herein, the term "prevention" refers to any action of inhibiting or delaying the onset of a disease by administration of a composition, and "treatment" refers to any action in which symptoms of a subject suspected of and suffering from a disease are improved or beneficially changed by administration of a composition.

Hereinafter, the present invention will be described in more detail through the examples, but the following examples are for illustrative purposes only and are not intended to limit the scope of the present invention.

### [Preparation Example]

The peptide of the present invention was synthesized based on the previously known solid phase peptide synthesis (SPPS) method, and the manufacturing process included the following steps.
Step 1: resin soaking and loading
Step 2: solid phase peptide synthesis (SPPS)
Step 3: deprotected peptide synthesis (global cleavage)
Step 4: primary purification and concentration / secondary purification and concentration
Step 5: freeze drying

The solid phase peptide synthesis (SPPS) method includes the following steps: loading a first amino acid into a resin, deprotecting the N-terminus of the amino acid with Fmoc, and then performing amino acid coupling according to the amino acid sequence, and synthesizing the protected peptide in a solid phase reactor. In addition, the amino acid coupling includes the following steps: loading a first amino acid into a resin and then removing Fmoc from the N-terminus of the amino acid; after completing the reaction, removing the solvent and washing the resin; coupling the next amino acid according to the sequence; after completing the reaction, removing the solvent and washing the resin; and repeating the above process until the final amino acid sequence was generated.

The alpha amine group of each amino acid is protected with a base-labile Fmoc group, and the functional group of the side chain is protected with an acid-labile group. All amino acids except Gly and Aib are in the L-configuration, and among them, several amino acids such as Hyp (tBu), Glu (tBu), and Gln (Trt) have unique protecting group. In addition, amino acids such as Ala, Leu, Aib, and Gly do not have a protecting group of the side chain. Therefore, after completing amino acid coupling according to the sequence, the resin and the protecting group were removed from the peptide to obtain a crude peptide. Thereafter, purification, concentration, and freeze-drying were performed to obtain the peptide compounds of Examples 1 to 45.

### [Test Example 1]

### Evaluation of the efficacy of the peptide to inhibit choroidal neovascularization: Measurement of fluorescence value (CTF; corrected total fluorescence) at the CNV site

In order to evaluate the efficacy of the peptide of the present invention to inhibit the choroidal neovascularization (CNV), an experiment was performed as follows.

The eyes of 7-8 week old C57BL/6 mice were instilled with a mydriatic agent to dilate the pupils, and the mice were anesthetized by intraperitoneal injection of ketamine (50 mg/kg) and rompun (23.32 mg/kg). A slit-lamp laser injection system was used to irradiate the laser to the choroid of each eye of the mouse four times in the 12 o'clock, 3 o'clock, 6 o'clock, and 9 o'clock directions. At this time, the laser irradiation conditions were 532 nm, 80 msec, and 240 mW. Immediately after laser irradiation, 1 µL of each peptide at a concentration of 10 mg/mL or aflibercept at a concentration of 20 mg/mL, which was dissolved in PBS, was injected into the vitreous body using a syringe. Ten days after drug administration, the mice were anesthetized, and 2% fluorescein was administered to the abdominal cavity to stain blood vessels. The retina was photographed 2 to 3 minutes after fluorescein administration. The size and fluorescence value of CNV lesions induced by laser were measured using the ImageJ program, and in order to reduce the differences between subjects, the CTF (corrected total fluorescence) was calculated by removing the background, and the values of each group were compared. The results of all tests are expressed as mean ± standard deviation, and significance was verified using one-way ANOVA and Kruskal-wallis test.

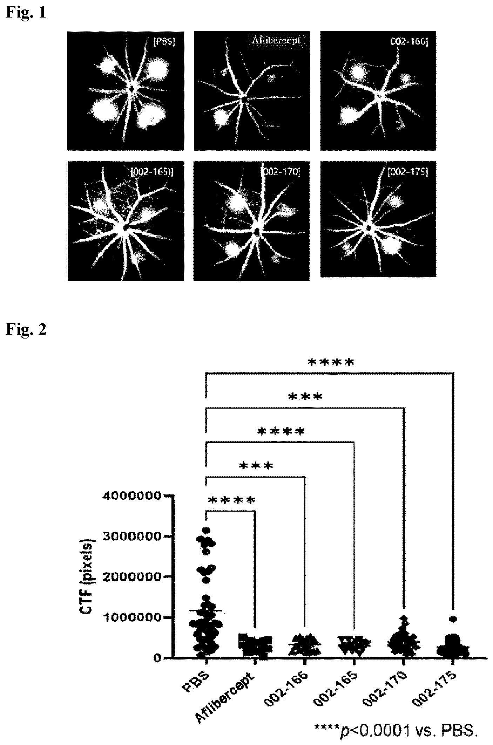

The results of the experiment are shown in Table 2 below and Figures 1 and 2.

**[Table 2]**

| Example | Peptide No. | Amino acid sequence | | | | | | | CTF* | |
|---|---|---|---|---|---|---|---|---|---|---|
| 1 | 002-053 | Hyp | Gly | Gln | Asp | Val | | | +++ | |
| 2 | 002-074 | Hyp | Gly | Gln | Asp | Leu | | | + | |
| 3 | 002-075 | Hyp | Gly | Gln | Asp | Ala | | | + | |
| 4 | 002-103 | Hyp | Gly | D-Gln | Asp | Gly | | | + | |
| 5 | 002-104 | Hyp | Gly | Gln | D-Asp | Gly | | | + | |
| 6 | 002-085 | D-Hyp | Gly | Gln | Asp | Gly | | | + | |
| 7 | 002-076 | Hyp | Gly | Gln | Asp | Val | Leu | | ++ | |
| 8 | 002-077 | Hyp | Gly | Gln | Asp | Leu | Leu | | ++ | |
| 9 | 002-078 | Hyp | Gly | Gln | Asp | Ala | Leu | | ++ | |
| 10 | 002-105 | Hyp | Gly | D-Gln | Asp | Gly | Leu | | + | |
| 11 | 002-106 | Hyp | Gly | Gln | D-Asp | Gly | Leu | | + | |
| 12 | 002-107 | Hyp | Gly | Gln | Asp | Gly | D-Leu | | + | |
| 13 | 002-090 | D-Hyp | Gly | Gln | Asp | Gly | Leu | | ++ | |
| 14 | 002-055 | Hyp | Gly | Gln | Asp | Val | Leu | Ala | ++ | |
| 15 | 002-080 | Hyp | Gly | Gln | Asp | Leu | Leu | Ala | ++ | |
| 16 | 002-081 | Hyp | Gly | Gln | Asp | Ala | Leu | Ala | + | |
| 17 | 002-084 | Hyp | Gly | Gln | Asp | Gly | Leu | Ala-IPE | ++ | |
| 18 | 002-108 | Hyp | Gly | D-Gln | Asp | Gly | Leu | Ala | + | |
| 19 | 002-109 | Hyp | Gly | Gln | D-Asp | Gly | Leu | Ala | + | |
| 20 | 002-110 | Hyp | Gly | Gln | Asp | Gly | D-Leu | Ala | + | |
| 21 | 002-111 | Hyp | Gly | Gln | Asp | Gly | Leu | D-Ala | + | |
| 22 | 002-086 | cis-4F-Pro | Gly | Gln | Asp | Gly | | | + | |
| 23 | 002-087 | trans-4NH2-Pro | Gly | Gln | Asp | Gly | | | ++ | |
| 24 | 002-088 | 4,4-difluoro-Pro | Gly | Gln | Asp | Gly | | | + | |
| 25 | 002-089 | 4-methylene-Pro | Gly | Gln | Asp | Gly | | | + | |
| 26 | 002-100 | 4,4-dimethyl Pro | Gly | Gln | Asp | Gly | | | + | |
| 27 | 002-091 | cis-4F-Pro | Gly | Gln | Asp | Gly | Leu | | +++ | |
| 28 | 002-092 | trans-4NH2-Pro | Gly | Gln | Asp | Gly | Leu | | + | |
| 29 | 002-093 | 4,4-difluoro-Pro | Gly | Gln | Asp | Gly | Leu | | + | |
| 30 | 002-094 | 4-methylene-Pro | Gly | Gln | Asp | Gly | Leu | | + | |
| 31 | 002-101 | 4,4-dimethyl | Gly | Gln | Asp | Gly | Leu | | + | |
| | | | Pro | | | | | | | |
| 32 | 002-095 | | D-Hyp | Gly | Gln | Asp | Gly | Leu | Ala | + |
| 33 | 002-096 | | cis-4F-Pro | Gly | Gln | Asp | Gly | Leu | Ala | + |
| 34 | 002-099 | | 4-methylene-Pro | Gly | Gln | Asp | Gly | Leu | Ala | + |
| 35 | 002-102 | | 4,4-dimethyl Pro | Gly | Gln | Asp | Gly | Leu | Ala | + |
| 36 | 002-130 | | Hyp | Gly | Gln | Glu | Gly | | | + |
| 37 | 002-170 | | Hyp | Gly | Gln | Glu | Val | | | + |
| 38 | 002-167 | | Hyp | Gly | Gln | Glu | Leu | Leu | | + |
| 39 | 002-168 | | Hyp | Gly | Gln | Glu | Val | Leu | | + |
| 40 | 002-132 | | Hyp | Gly | Gln | Glu | Gly | Leu | Ala | + |
| 41 | 002-165 | | Hyp | Gly | Gln | Glu | Leu | Leu | Ala | + |
| 42 | 002-166 | | Hyp | Gly | Gln | Glu | Val | Leu | Ala | + |
| 43 | 002-175 | | Hyp | Gly | Gln | Glu | Aib | Leu | Ala | ++ |
| 44 | 002-176 | | Hyp | Gly | Gln | Glu | tert-Leu | Leu | Ala | + |
| 45 | 002-177 | | Hyp | Gly | Gln | Glu | phenyl-Gly | Leu | Ala | + |
| PBS | | | | | | | | | | - |
| aflibercept | | | | | | | | | | + |
| *CTF value | | + | : 300,000 to 800,000 | | | | | | | |
| | | ++ | : 200,000 to 300,000 | | | | | | | |
| | | +++ | : 100,000 to 200,000 | | | | | | | |

As shown in Table 2 above and Figures 1 and 2, as a result of measuring the fluorescence value of the CNV site, it was confirmed that the peptide of the present invention has a CNV inhibitory effect, and in particular, the peptides of Example 1 (002-053), Example 7 (002-076), Example 8 (002-077), Example 9 (002-078), Example 13 (002-090), Example 14 (002-055), Example 15 (002-080), Example 17 (002-084), Example 23 (002-087), Example 27 (002-091), and Example 43 (002-175) showed very excellent inhibitory efficacy.

Therefore, it can be seen that the peptide of the present invention has excellent CNV inhibitory efficacy.

### [Test Example 2]

### Evaluation of the efficacy of the peptide to inhibit choroidal neovascularization: optical coherence tomography

In order to evaluate the efficacy of the peptide of the present invention to inhibit the choroidal neovascularization (CNV), an experiment was performed as follows.

The eyes of 7-8 week old C57BL/6 mice were instilled with a mydriatic agent to dilate the pupils, and the mice were anesthetized by intraperitoneal injection of ketamine (50 mg/kg) and rompun (23.32 mg/kg). A slit-lamp laser injection system was used to irradiate the laser to the choroid of each eye of the mouse four times in the 12 o'clock, 3 o'clock, 6 o'clock, and 9 o'clock directions. At this time, the laser irradiation conditions were 532 nm, 80 msec, and 240 mW. Immediately after laser irradiation, 1 µL of each peptide at a concentration of 10 mg/mL or aflibercept at a concentration of 20 mg/mL, which was dissolved in PBS, was injected into the vitreous body using a syringe. Ten days after drug administration, the mice were anesthetized, and the CNV lesions induced by laser were calculated and expressed as µm² using the ImageJ program by transmitting an OCT beam for each burn. The results of all tests are expressed as mean ± standard deviation, and significance was verified using one-way ANOVA and Kruskal-wallis test.

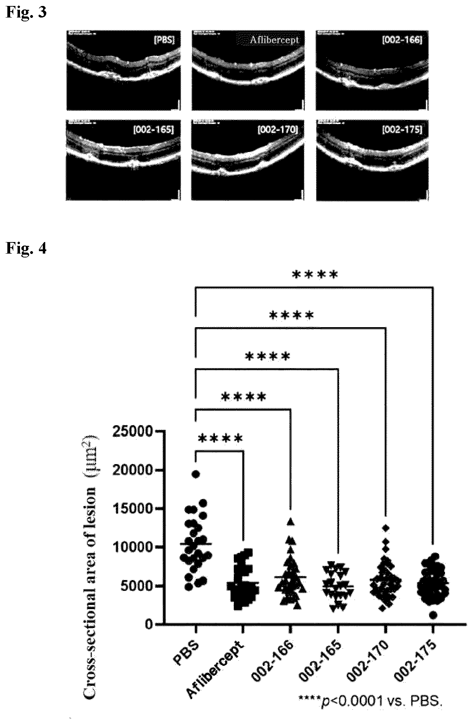

The results of the experiment are shown in Table 3 below and Figures 3 and 4.

**[Table 3]**

| Example | Peptide No. | Amino acid sequence | | | | | | | Size of lesion (µm²) |
|---|---|---|---|---|---|---|---|---|---|
| 1 | 002-053 | Hyp | Gly | Gln | Asp | Val | | | 5,636 |
| 3 | 002-075 | Hyp | Gly | Gln | Asp | Ala | | | 6,491 |
| 7 | 002-076 | Hyp | Gly | Gln | Asp | Val | Leu | | 6,046 |
| 13 | 002-090 | D-Hyp | Gly | Gln | Asp | Gly | Leu | | 6,395 |
| 14 | 002-055 | Hyp | Gly | Gln | Asp | Val | Leu | Ala | 5,407 |
| 15 | 002-080 | Hyp | Gly | Gln | Asp | Leu | Leu | Ala | 6,672 |
| 17 | 002-084 | Hyp | Gly | Gln | Asp | Gly | Leu | Ala-IPE | 5,978 |
| 34 | 002-099 | 4-methylene-Pro | Gly | Gln | Asp | Gly | Leu | Ala | 8,603 |
| 37 | 002-170 | Hyp | Gly | Gln | Glu | Val | | | 5,775 |
| 41 | 002-165 | Hyp | Gly | Gln | Glu | Leu | Leu | Ala | 4,960 |
| 42 | 002-166 | Hyp | Gly | Gln | Glu | Val | Leu | Ala | 6,102 |
| 43 | 002-175 | Hyp | Gly | Gln | Glu | Aib | Leu | Ala | 5,393 |
| PBS | | | | | | | | | 10,428 |
| aflibercept | | | | | | | | | 5,437 |

As shown in Table 3 above and Figures 3 and 4, as a result of measuring the size of the CNV lesion, it was confirmed that the size of the CNV lesion was reduced when the peptide of the present invention was injected. Therefore, it can be seen that the peptide of the present invention has excellent CNV inhibitory efficacy.

### [Test Example 3]

### Evaluation of the efficacy of the peptide to inhibit choroidal neovascularization: electroretinography

In order to evaluate the efficacy of the peptide of the present invention to inhibit the choroidal neovascularization (CNV), an experiment was performed as follows.

The eyes of 7-8 week old C57BL/6 mice were instilled with a mydriatic agent to dilate the pupils, and the mice were anesthetized by intraperitoneal injection of ketamine (50 mg/kg) and rompun (23.32 mg/kg). A slit-lamp laser injection system was used to irradiate the laser to the choroid of each eye of the mouse four times in the 12 o'clock, 3 o'clock, 6 o'clock, and 9 o'clock directions. At this time, the laser irradiation conditions were 532 nm, 80 msec, and 240 mW. Immediately after laser irradiation, 1 µL of each peptide at a concentration of 10 mg/mL or aflibercept at a concentration of 20 mg/mL, which was dissolved in PBS, was injected into the vitreous body using a syringe. For electroretinogram analysis, dark adaptation was performed for more than 12 hours. The mice were subjected to mydriasis in a dark room and anesthetized, and then electroretinography was performed by contacting the ground electrode to the tail, the reference electrode to the head, and the corneal electrode to the cornea. The response values were obtained by stimulating the retina with a single white light of 0.9 cd·sec/m² flash intensity, and the amplitude, which is determined from the trough of the α-wave to the peak of the b-wave, was measured and evaluated as an indicator of retinal function. The results of all tests are expressed as mean ± standard deviation, and significance was verified using one-way ANOVA and Kruskal-wallis test.

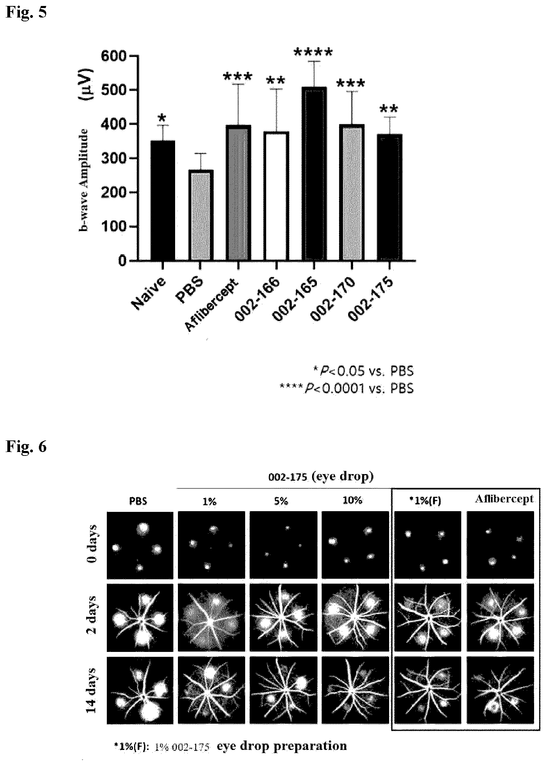

The results of the experiment are shown in Table 4 below and Figure 5.

**[Table 4]**

| Example | Peptide No. | Amino acid sequence | | | | | | | b-wave amplitude (µV) |
|---|---|---|---|---|---|---|---|---|---|
| 37 | 002-170 | Hyp | Gly | Gln | Glu | Val | | | 400 |
| 41 | 002-165 | Hyp | Gly | Gln | Glu | Leu | Leu | Ala | 510 |
| 42 | 002-166 | Hyp | Gly | Gln | Glu | Val | Leu | Ala | 380 |
| 43 | 002-175 | Hyp | Gly | Gln | Glu | Aib | Leu | Ala | 371 |
| Naive | | | | | | | | | 353 |
| PBS | | | | | | | | | 240 |
| aflibercept | | | | | | | | | 398 |

As shown in Table 4 above and Figure 5, as a result of measuring the b-wave amplitude, it was confirmed that the b-wave amplitude was increased when the peptide of the present invention was injected. Therefore, it can be seen that the peptide of the present invention has excellent CNV inhibitory efficacy.

### [Test Example 4]

### Evaluation of the stability of the peptide

In order to evaluate the stability of the peptide of the present invention, an experiment was performed as follows.

The stability of the peptide was confirmed through content analysis in an aqueous solution at 40°C, 75% humidity, and harsh conditions, and was analyzed using HPLC under the following conditions.
Analysis column: C18 (4.6 Y 250 mm, 5 µm, YMC Hydrosphere)
Analysis wavelength: ultraviolet absorption spectrophotometer (210 nm)
Injection volume / Flow rate: 10 µL / 0.8 mL/min
Column temperature: 45 °C
Mobile phase: 0.1 M phosphate buffer methanol mixture (83:17)

The results of the experiment are shown in Table 5 below.

**[Table 5]**

| Example | Peptide No. | Initial content (%) | Content after 7 days (%) | Decrease rate (%) |
|---|---|---|---|---|
| 40 | 002-132 | 96.123 | 95.685 | 0.438 |
| 41 | 002-165 | 98.155 | 97.555 | 0.600 |
| 43 | 002-175 | 96.917 | 96.562 | 0.355 |
| 44 | 002-176 | 98.390 | 97.785 | 0.605 |
| 45 | 002-177 | 98.586 | 98.147 | 0.439 |

As shown in Table 5 above, as a result of evaluating the stability, it was confirmed that the content of all peptides of the present invention was maintained at about 95% or more after 7 days, and the decrease rate after 7 days was also less than about 1%, indicating that the stability was very excellent. In particular, the peptide of Example 43 (002-175) showed the most excellent stability. Therefore, it can be seen that the peptide of the present invention has excellent stability.

### [Test Example 5]

### Evaluation of the solubility of the peptide

In order to evaluate the solubility of the peptide of the present invention, an experiment was performed as follows.

The solubility test was conducted based on the Korean Pharmacopoeia method, and the solubility was determined as the degree to which 0.1 g of peptide was dissolved within 30 minutes when added to 10 mL of water and shaken vigorously for 30 seconds every 5 minutes at 20 ± 5 °C.

The results of the experiment are shown in Table 6 below.

**[Table 6]**

| Example | Peptide No. | Solubility (mg/ml) |
|---|---|---|
| 40 | 002-132 | 330.0 |
| 43 | 002-175 | 500.0 |
| 44 | 002-176 | 100.0 |

As shown in Table 6 above, as a result of measuring the solubility, the peptide of the present invention showed excellent solubility, and in particular, the peptide of Example 43 (002-175) showed the most excellent solubility. Therefore, it can be seen that the peptide of the present invention has excellent solubility.

### [Test Example 6]

### Evaluation of the efficacy of an eye drop comprising the peptide to inhibit choroidal neovascularization

In order to evaluate the efficacy of the eye drop comprising the peptide of Example 43 (002-175), which was confirmed to be an excellent peptide through the above test examples, to inhibit the choroidal neovascularization (CNV), an experiment was performed as follows.

The eyes of 7-8 week old C57BL/6 mice were instilled with a mydriatic agent to dilate the pupils, and the mice were anesthetized by intraperitoneal injection of ketamine (50 mg/kg) and rompun (23.32 mg/kg). A slit-lamp laser injection system was used to irradiate the laser to the choroid of each eye of the mouse four times in the 12 o'clock, 3 o'clock, 6 o'clock, and 9 o'clock directions. At this time, the laser irradiation conditions were 532 nm, 80 msec, and 240 mW. Three days after laser irradiation and before drug administration, 1 µL of aflibercept at a concentration of 20 mg/mL, which was dissolved in PBS, was injected into the vitreous body, respectively, using a syringe, and the drugs prepared at different concentrations were instilled in both eyes twice a day (5 µL/eye, AM: 9:00 and PM 04:00) for 11 days. Six, ten, and fourteen days after laser induction, the mice were anesthetized, and 2% fluorescein was administered to the abdominal cavity to stain blood vessels. The retina was photographed 2 to 3 minutes after fluorescein administration. The size and fluorescence value of CNV lesions induced by laser were measured using the ImageJ program, and in order to reduce the differences between subjects, the CTF (corrected total fluorescence) was calculated by removing the background, and the values of each group were compared. In addition, six, ten, and fourteen days after laser induction, the mice were anesthetized, and the CNV lesions induced by laser were calculated and expressed as µm² using the ImageJ program by transmitting an OCT beam for each burn. The results of all tests are expressed as mean ± standard deviation, and significance was verified using one-way ANOVA and Kruskal-wallis test, and the results of the experiment are shown in Figures 6 to 9.

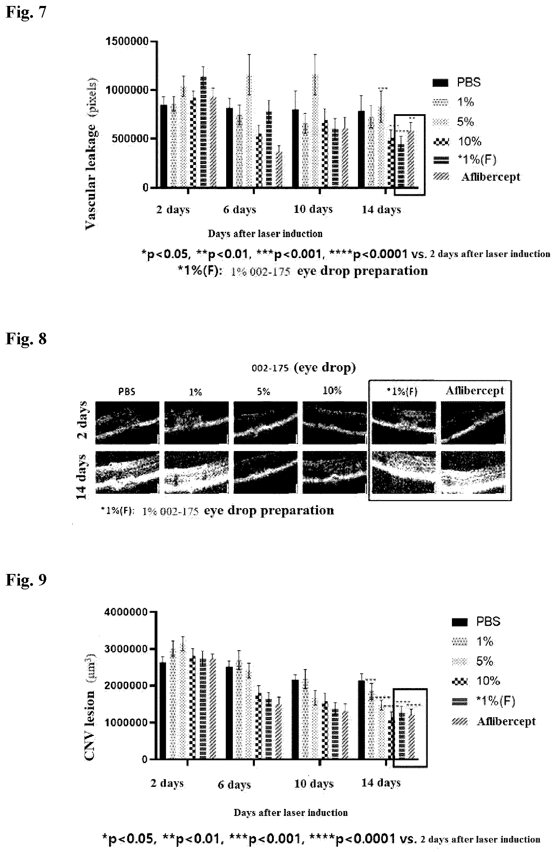

As shown in Figures 6 and 7, as a result of measuring the fluorescence value of the CNV site, the eye drop comprising the peptide of Example 43 (002-175) of the present invention showed excellent CNV inhibitory efficacy, and in particular, the eye drop showed more excellent inhibitory efficacy compared to aflibercept, a positive control group, after 14 days of administration.

In addition, as shown in Figures 8 and 9, as a result of measuring the size of the CNV lesion, it was confirmed that the size of the CNV lesion was reduced when the eye drop comprising the peptide of Example 43 (002-175) of the present invention was administered, and in particular, the size was reduced to a similar level compared to aflibercept, a positive control group.

Therefore, it can be seen that the eye drop comprising the peptide of Example 43 (002-175) of the present invention has excellent CNV inhibitory efficacy.

### [Test Example 7]

### Evaluation of the efficacy of an eye drop comprising the peptide to inhibit neovascularity

A symptom of neovascular macular degeneration is retinal angiomatous proliferation (RAP), which occurs in approximately 12 to 15% of patients who are newly diagnosed with neovascular macular degeneration. Meanwhile, *Vldlr* is known to be one of the functional candidate genes for human macular degeneration, and mice in which *Vldlr* is knocked out (-/-) show retinal hemangioma proliferation along with subretinal neovascularization. Therefore, in order to evaluate the neovascularity inhibitory efficacy of the eye drop comprising the peptide of Example 43 (002-175), which was confirmed to be an excellent peptide through the above test examples, in *Vldlr* knockout (-/-) mice, an animal model of neovascular macular degeneration disease, an experiment was performed as follows.

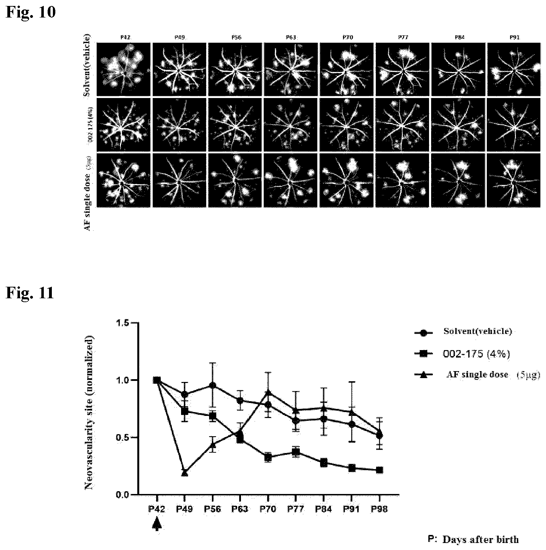

From 6 weeks of age (P42) to 14 weeks of age (P98), Vldlr ^{-/-} mice were instilled with the eye drop comprising the peptide of Example 43 (002-175) twice a day, and as a positive control group, aflibercept at a concentration of 20 mg/mL was intraocularly injected once, 1 µL each, into the vitreous body of 6 week old mice. The eyes of the mice were instilled with a mydriatic agent to dilate the pupils, and then the mice were anesthetized by intraperitoneal injection of ketamine (50 mg/kg) and rompun (23.32 mg/kg), and 2% fluorescein was injected to the abdominal cavity to stain blood vessels. Images were acquired through retinal fluorescence imaging, and the lesion area was calculated using the ImageJ program, and the results of the experiment are shown in Figures 10 and 11.

As shown in Figures 10 and 11, as a result of calculating the area of new blood vessels, the size of the lesion in the group administered with aflibercept (AF), which is a positive control group, was reduced rapidly 1 week after administration (P49), but the size of the lesion was gradually increased again from the 2nd week and reached a size similar to that of the group administered with vehicle, a negative control group, after 8 weeks (P98). On the other hand, in the group administered with the eye drop comprising the peptide of Example 43 (002-175) of the present invention the size of the lesion was gradually reduced, and the size of the lesion was shown to be suppressed after 8 weeks of administration (P98).

Therefore, it can be seen that the eye drop comprising the peptide of Example 43 (002-175) of the present invention has excellent neovascularity inhibitory efficacy.

## Claims

1. A pharmaceutical composition for preventing or treating macular degeneration, comprising a peptide represented by the following formula 1:
[Formula 1] X₁-X₂-X₃-X₄-X₅-X₆-X₇
in the formula, X₁ is an amino acid selected from the group consisting of proline, trans-4-hydroxy-L-proline, trans-4-hydroxy-D-proline, cis-4-fluoro-L-proline, 4,4,-difluoro-L-proline, 4-methylene-L-proline, 4,4-dimethyl-L-proline, and trans-4-amino-L-proline,
X₂ is glycine,
X₃ is glutamine or D-glutamine,
X₄ is an amino acid selected from the group consisting of aspartate, D-aspartate, glutamate, and D-glutamate,
X₅ is a non-polar amino acid,
X₆ is absent, or leucine or D-leucine, and
X₇ is absent, or an amino acid selected from the group consisting of alanine, D-alanine, and alanine-isopropyl ester,
wherein when X₆ is absent, X₇ is also absent.

2. The pharmaceutical composition for preventing or treating macular degeneration according to claim 1, wherein
in the formula, X₁ is an amino acid selected from the group consisting of proline, trans-4-hydroxy-L-proline, trans-4-hydroxy-D-proline, cis-4-fluoro-L-proline, 4,4,-difluoro-L-proline, 4-methylene-L-proline, 4,4-dimethyl-L-proline, and trans-4-amino-L-proline,
X₂ is glycine,
X₃ is glutamine or D-glutamine,
X₄ is an amino acid selected from the group consisting of aspartate, D-aspartate, glutamate, and D-glutamate,
X₅ is an amino acid selected from the group consisting of glycine, phenyl-glycine, alanine, valine, leucine, tert-leucine, and 2-aminoisobutyric acid,
X₆ is absent, or leucine or D-leucine, and
X₇ is absent, or an amino acid selected from the group consisting of alanine, D-alanine, and alanine-isopropyl ester.

3. The pharmaceutical composition for preventing or treating macular degeneration according to claim 1, wherein
in the formula, X₁ is an amino acid selected from the group consisting of proline, trans-4-hydroxy-L-proline, trans-4-hydroxy-D-proline, cis-4-fluoro-L-proline, 4,4,-difluoro-L-proline, 4-methylene-L-proline, 4,4-dimethyl-L-proline, and trans-4-amino-L-proline,
X₂ is glycine,
X₃ is glutamine or D-glutamine,
X₄ is an amino acid selected from the group consisting of aspartate, D-aspartate, glutamate, and D-glutamate,
X₅ is a non-polar amino acid,
X₆ is leucine or D-leucine, and
X₇ is an amino acid selected from the group consisting of alanine, D-alanine, and alanine-isopropyl ester.

4. The pharmaceutical composition for preventing or treating macular degeneration according to claim 1, wherein
in the formula, X₁ is an amino acid selected from the group consisting of proline, trans-4-hydroxy-L-proline, trans-4-hydroxy-D-proline, cis-4-fluoro-L-proline, 4,4,-difluoro-L-proline, 4-methylene-L-proline, 4,4-dimethyl-L-proline, and trans-4-amino-L-proline,
X₂ is glycine,
X₃ is glutamine or D-glutamine,
X₄ is an amino acid selected from the group consisting of aspartate, D-aspartate, glutamate, and D-glutamate,
X₅ is an amino acid selected from the group consisting of glycine, phenyl-glycine, alanine, valine, leucine, tert-leucine, and 2-aminoisobutyric acid,
X₆ is leucine or D-leucine, and
X₇ is an amino acid selected from the group consisting of alanine, D-alanine, and alanine-isopropyl ester.

5. The pharmaceutical composition for preventing or treating macular degeneration according to claim 1, wherein
in the formula, X₁ is an amino acid selected from the group consisting of proline, trans-4-hydroxy-L-proline, trans-4-hydroxy-D-proline, cis-4-fluoro-L-proline, 4,4,-difluoro-L-proline, 4-methylene-L-proline, 4,4-dimethyl-L-proline, and trans-4-amino-L-proline,
X₂ is glycine,
X₃ is glutamine or D-glutamine,
X₄ is glutamate or D-glutamate,
X₅ is 2-aminoisobutyric acid,
X₆ is leucine or D-leucine, and
X₇ is an amino acid selected from the group consisting of alanine, D-alanine, and alanine-isopropyl ester.

6. The pharmaceutical composition for preventing or treating macular degeneration according to claim 1, wherein the pharmaceutical composition comprises any one peptide selected from the group consisting of the amino acid sequences of SEQ ID NOs: 1 to 45.

7. The pharmaceutical composition for preventing or treating macular degeneration according to claim 1, wherein the pharmaceutical composition comprises a peptide consisting of the amino acid sequence of SEQ ID NO: 43.

8. The pharmaceutical composition for preventing or treating macular degeneration according to any one of claims 1 to 7, wherein the peptide inhibits choroidal neovascularization (CNV).

9. The pharmaceutical composition for preventing or treating macular degeneration according to claim 1, wherein the macular degeneration is at least one selected from the group consisting of wet macular degeneration, dry macular degeneration, and age-related macular degeneration.
